# EUROPEAN PATENT SPECIFICATION

(11) **EP 4 253 425 B1**
(45) Date of publication and mention of the grant of the patent: **14.10.2026**
(21) Application number: 21898048.0
(22) Date of filing: 25.11.2021
(51) Int. Cl.: C08B 11/12, A61Q 9/02, A61Q 11/00, A61Q 19/10, C08L 1/26, A61K 8/02, A61K 8/36, A61K 8/42, A61K 8/44, A61K 8/46, A61K 8/55, A61K 8/73, A61K 8/84, A23L 29/00, A23L 29/10, A23L 29/231, A23L 29/244, A23L 29/262, C09K 23/00

(54) **ADDITIVE FOR USE IN PRODUCTION OF AIR BUBBLE-CONTAINING COMPOSITION**
ADDITIV ZUR VERWENDUNG BEI DER HERSTELLUNG EINER LUFTBLASENHALTIGEN ZUSAMMENSETZUNG
ADDITIF DESTINÉ À ÊTRE UTILISÉ EN PRODUCTION D'UNE COMPOSITION CONTENANT DES BULLES D'AIR

(30) Priority: 27.11.2020 JP 2020196554
(43) Date of publication of application: 04.10.2023
(73) Proprietor: Nippon Paper Industries Co., Ltd., Tokyo 114-0002 (JP)
(72) Inventor: MIYATA, Sonoka, Tokyo 114-0002 (JP); TADA, Yusuke, Tokyo 114-0002 (JP); SATO, Shinji, Tokyo 114-0002 (JP); FUNATSU, Kei, Tokyo 114-0002 (JP)
(74) Representative: Carpmaels & Ransford LLP
(86) International application number: PCT/JP2021/043260
(87) International publication number: WO 2022/114076

(56) References cited:
- EP-A1- 3 546 640
- WO-A1-2014/088072
- WO-A1-2017/217511
- JP-A- 2002 145 720
- JP-A- 2008 238 115
- JP-A- 2015 149 929
- JP-A- 2015 199 699
- JP-A- 2017 066 283
- JP-A- 2017 066 283
- JP-A- 2018 104 624
- JP-A- 2019 206 521
- JP-A- 2019 206 521
- JP-A- 2019 206 607
- JP-A- 2019 505 576
- JP-A- 2020 150 939

## Description

### TECHNICAL FIELD

The present invention relates to the use of an additive for producing air bubble-containing compositions which is added in producing an air bubble-containing composition, or to the air bubble-containing composition to promote foaming and improve the stability of foam formed.

### BACKGROUND ART

There are conventionally used cellulose-based materials such as carboxymethylcellulose, as additives which form fine creamy foam and further holds good the dispersed state of air bubbles in the foam and improves the foam stability in foods, cosmetics, detergents (solid soaps, kitchen detergents, body soaps, shampoos, tooth powders and the like), resins, agrochemicals, civil engineering materials, boring materials (foam shielding materials and the like), and the like, which are intended to contain foam or to cause foaming in use.

For example, PTL 1 discloses use of a carboxymethylcellulose·sodium salt with 0.8 to 2.0 in degree of etherification and 1 to 30 mPa·s in viscosity of a 2% aqueous solution, as a stabilizer for ice cream with low air content. PTL 2 describes that by blending, in a liquid egg, a crystalline fibrous cellulose, and/or a cellulose-based dry composition containing 30 to 95% by mass of the fibrous cellulose and 5 to 70% by mass of a water-soluble polymer and/or a hydrophilic substance, the foam stability and the like of a processed egg food is improved.

PTL 3 describes use of chemically modified cellulose nanofibers as an additive for an air bubble-containing composition. PTL 4 and 5 disclose the combination of CMC with surfactants or PEG for improving the foaming and foam stability.

### CITATION LIST

### PATENT LITERATURE

PTL 1: Japanese Patent Laid-Open No. 2005-143321
PTL 2: Japanese Patent Laid-Open No. 2009-065836
PTL 3: Japanese Patent Laid-Open No. 2017-66283
PTL4: European Patent Application EP 3546640 A1
PTL5: International Patent Applciation WO 2017217511 A1

### SUMMARY OF INVENTION

### TECHNICAL PROBLEM

The method disclosed in PTL 1 which uses a water-soluble carboxymethylcellulose. and the method disclosed in PTL 2 which uses a chemically unmodified fibrous cellulose are not sufficient in the persistency of foam (foam stability). By contrast, in the method using chemically modified cellulose nanofibers as described in PTL 3, the foam stabilizing effect can be attained. However, it has now been found that when chemically modified cellulose nanofibers alone are used as an additive, the foaming performance (power of initial foam formation) is rather inferior. The present invention has an object to further improve the method described in PTL 3 and provide the use of an additive for producing air bubble (foam)-containing compositions, the additive retaining the foam stability and simultaneously having high foaming performance (power of foam formation).

### SOLUTION TO PROBLEM

As a result of exhaustive studies on the above problem, the present inventors have found that foam stability of a foam (air bubble)-containing composition is retained, and simultaneously a foaming performance can be enhanced by using carboxymethylated cellulose nanofibers having a specific degree of carboxymethyl substitution and a specific degree of crystallinity of cellulose type I together with a nonionic water-soluble polymer to prepare an additive for use in the production of the composition. The present invention is not limited thereto, but includes the following.
[1] Use of an additive for producing an air bubble-containing composition, the additive comprising:
   carboxymethylated cellulose nanofibers having a degree of carboxymethyl substitution per glucose unit of 0.01 to 0.50 and a degree of crystallinity of cellulose type I of 40% or higher, and
   a nonionic water-soluble polymer, wherein the nonionic water-soluble polymer is at least one selected from hydroxyethylcellulose, methylcellulose, hydroxypropylcellulose, hydroxypropylmethylcellulose, or hydroxyethylmethylcellulose.
[2] The use according to [1], wherein the amount of the nonionic water-soluble polymer is 1 to 5,000 parts by mass with respect to 100 parts by mass of the carboxymethylated cellulose nanofibers.
[3] A method for producing an air bubble-containing composition, the method including mixing and stirring the additive defined in [1] or [2] into materials for the air bubble-containing composition to cause foaming of the materials.
[4] A method for stabilizing air bubbles in an air bubble-containing composition, including adding the additive defined in [1] or [2] to the air bubble-containing composition.

### ADVANTAGEOUS EFFECTS OF INVENTION

The additive used in the present invention can be added when producing an air bubble-containing composition to enhance the foaming performance of the composition and also the persistency (foam stability) of foam formed. In addition, the additive can be added to the air bubble (foam)-containing composition to promote further foaming and impart foam stability. That is, the additive used in the present invention can impart both of foaming performance and foam stability to a composition that is intended to contain air bubbles (foam). The additive used in the present invention for producing an air bubble-containing composition contains specific carboxymethylated cellulose nanofibers, and a nonionic water-soluble polymer as defined in the claims. If, for example, an anionic water-soluble polymer is used in place of the nonionic water-soluble polymer, the above effect cannot be attained as indicated in Comparative Examples 1 to 3. The additive used in the present invention may contain specific carboxymethylated cellulose nanofibers, and a nonionic water soluble polymer as defined in the claims, and a surfactant.

The additive used in the present invention for producing an air bubble-containing composition can be used suitably, for example, in foods and beverages (ice cream, dough for cakes, hot cakes and the like, bread, whipped cream, and the like), cosmetics (facial wash, shaving cream and the like), detergents (solid soaps, kitchen detergents, body soaps, shampoos, tooth powders and the like), resins, agrochemicals, civil engineering materials, boring materials (foam shielding materials and the like), etc., which are intended to contain foam or to cause foaming in use.

### BRIEF DESCRIPTION OF DRAWINGS

[Fig. 1]Fig. 1 is photographs showing results of Example 1.
[Fig. 2]Fig. 2 is photographs showing results of Reference Example 2.
[Fig. 3]Fig. 3 is photographs showing results of Comparative Example 1.
[Fig. 4]Fig. 4 is photographs showing results of Comparative Example 2.
[Fig. 5]Fig. 5 is photographs showing results of Comparative Example 3.
[Fig. 6]Fig. 6 is photographs showing results of Reference Example 3.
[Fig. 7]Fig. 7 is photographs showing results of Example 4.
[Fig. 8]Fig. 8 is photographs showing results of Reference Example 5.
[Fig. 9]Fig. 9 is photographs showing results of Reference Example 6.
[Fig. 10]Fig. 10 is photographs showing results of Reference Example 7.
[Fig. 11]Fig. 11 is photographs showing results of Reference Example 8.

### DESCRIPTION OF EMBODIMENTS

### <Carboxymethylated cellulose nanofibers>

In the present invention, carboxymethylated cellulose nanofibers (hereinafter, "carboxymethylated" may be abbreviated as "CM-"; and cellulose nanofibers as "CNFs") is obtained by finely dividing a carboxymethylated cellulose (CM-cellulose) to a fiber width on a nanometer level, and are usually fine fibers with a fiber width of about 3 to several hundreds of nanometers, for example, about 4 to 500 nm. The aspect ratio is not limited, but is, for example, 50 or higher and preferably 100 or higher. The average fiber diameter and the average fiber length of the CM-CNFs can be determined by using an atomic force microscope (AFM), a transmission electron microscope (TEM) or a field emission scanning electron microscope (FE-SEM), and observing 200 fibers randomly selected and calculating an average value of fiber diameters and fiber lengths obtained by the observation result. Then, the aspect ratio can be calculated by dividing the average fiber length by the average fiber diameter. The CM-CNFs can be obtained by applying a mechanical force to the CM-cellulose to finely divide (defibrate) the cellulose.

The CM-cellulose has a structure in which part of hydroxyl groups in glucose residues constituting a cellulose molecule are ether bonded with carboxymethyl groups. The CM-cellulose may assume a salt form, and the CM-cellulose referred to in the present description includes a salt of the CM-cellulose. Examples of salts of the CM-cellulose include metal salts, such as sodium salt, of the CM-cellulose.

### <Cellulose raw material>

Examples of cellulose raw materials for producing the CM-cellulose that is to be a CM-CNF include plant-based materials (for example, wood, bamboo, hemp, jute, kenaf, farmland residual waste, cloth, pulp (softwood unbleached kraft pulp (NUKP), softwood bleached kraft pulp (NBKP), hardwood unbleached kraft pulp (LUKP), hardwood bleached kraft pulp (LBKP), softwood unbleached sulfite pulp (NUSP), softwood bleached sulfite pulp (NBSP), thermomechanical pulp (TMP), regenerated pulp, waste paper and the like), animal materials (for example, ascidians), algae, microbes (for example, acetobacter), and materials originated from products of microbes, and any thereof can be used. Preferably, the materials are cellulose fibers originated from plants or microbes, and more preferably, the materials are cellulose fibers originated from plants.

### <Carboxymethylation of the cellulose raw material>

The CM-cellulose that is a raw material for the CM-CNF may be obtained by carboxymethylating the above cellulose raw material by a well-known method, or a commercially available product may be used. In either case, the CM-cellulose preferably has a degree of carboxymethyl group substitution of 0.01 to 0.50 per anhydrous glucose unit of the cellulose. An example of methods for producing such a CM-cellulose can be the following method.

As a solvent, water and/or a lower alcohol, specifically, water, methanol, ethanol, n-propyl alcohol, isopropyl alcohol, n-butanol, isobutanol, tertiary butanol or the like, singly, or a mixed medium of two or more kinds thereof, is used in 3 to 20 mass times the cellulose raw material. As a mercerizing agent, an alkali metal hydroxide, specifically sodium hydroxide or potassium hydroxide, is used in 0.5 to 20 mol times per anhydrous glucose residue of the cellulose raw material. A mercerization treatment is carried out by mixing the cellulose raw material, the solvent, and the mercerizing agent, at a reaction temperature of 0 to 70°C, preferably 10 to 60°C, for a reaction time of 15 min to 8 hours, preferably 30 min to 7 hours. Thereafter, an etherification reaction is carried out by adding a carboxymethylating agent in 0.05 to 10.0 mol times per glucose residue, at a reaction temperature of 30 to 90°C, preferably 40 to 80°C, and for a reaction time of 30 min to 10 hours, preferably 1 hour to 4 hours.

The CM-cellulose that is a raw material for the CM-CNF retains at least a part of the fibrous shape even when it is dispersed in water, and is differentiated from a carboxymethyl cellulose which is one kind of a water-soluble polymer described later. When a water dispersion of the "carboxymethylated cellulose (CM-cellulose) is observed by an electron microscope, a fibrous substance can be observed. By contrast, even if a water dispersion of a carboxymethyl cellulose which is one kind of a water-soluble polymer is observed, no fibrous substance is observed. Further, in X-ray diffraction measurement, although the "carboxymethylated cellulose" has a peak of the cellulose type I crystal observed, the carboxymethyl cellulose being a water-soluble polymer has no cellulose type I crystal observed.

### <Defibration of the CM-cellulose>

By defibrating the CM-cellulose, the CM-CNF can be produced. An apparatus to be used for defibration is not particularly limited, but there can be used an apparatus such as of a high-speed rotation type, a colloid mill type, a high-pressure type, a roll mill type or an ultrasonic type. In the defibration, it is preferable to apply a strong shearing force to a dispersion of the CM-cellulose. In particular, in order to efficiently carry out the defibration, it is preferable to use a wet high-pressure or ultrahigh-pressure homogenizer which can apply a pressure of 50 MPa or higher to the dispersion and can apply a strong shearing force thereto. The pressure is more preferably 100 MPa or higher and still more preferably 140 MPa or higher. Then, prior to the defibration and the dispersing treatment by the high-pressure homogenizer, the dispersion may be subjected to a pretreatment using a known mixing, stirring, emulsifying and dispersing apparatus such as a high-speed shearing mixer, if necessary.

The solid content of the dispersion of the CM-cellulose to be provided for the defibration is, from the viewpoint of the treatment efficiency, preferably 0.1% by mass or higher, more preferably 0.2% by mass or higher, and particularly preferably 0.3% by mass or higher. Further from the viewpoint of the flowability, the content is preferably 10% by mass or lower and more preferably 6% by mass or lower.

### <Degree of carboxymethyl substitution>

In the CM-CNF, the degree of carboxymethyl substitution per anhydrous glucose unit of the cellulose is 0.01 to 0.50. If the degree of carboxymethyl substitution is lower than 0.01, sufficient foam stability and foaming performance cannot be attained. On the other hand, if the degree of carboxymethyl substitution exceeds 0.50, the dissolution of cellulose in an aqueous medium becomes liable to occur and the shape of fiber becomes unable to be retained and the foam stability reduces. The minimum value of the degree of carboxymethyl substitution is more preferably 0.10 or higher and still more preferably 0.20 or higher. The maximum value of the degree of carboxymethyl substitution is more preferably 0.40 or lower. The degree of carboxymethyl substitution of the CM- CNF can be regulated by controlling the amount of the carboxymethylating agent to be added for reaction in producing the CM-cellulose that is a raw material, the amount of the mercerizing agent, and the composition ratio of water and the organic solvent. The degree of carboxymethyl substitution of the CM-cellulose and the degree of carboxymethyl substitution of the CM-CNFs obtained by defibrating the CM-cellulose are usually the same.

In the present description, the anhydrous glucose unit means an individual anhydrous glucose (glucose residue) constituting the cellulose. Further, the degree of carboxymethyl substitution (referred to also as degree of etherification) indicates the proportion of hydroxyl groups replaced by carboxymethyl ether groups among hydroxyl groups in glucose residues constituting the cellulose (the number of carboxymethyl ether groups per one glucose residue). Here, the degree of carboxymethyl substitution may be abbreviated as DS.

A measuring method of the degree of carboxymethyl substitution is as follows.

About 2.0 g of a sample is precisely weighed and put in a stoppered 300-mL conical flask. 100 mL of nitric acid methanol (a solution in which 100 mL of a guaranteed concentrated nitric acid is added to 1,000 mL of methanol) is added, and shaken for 3 hours to convert a salt of a CM-cellulose (CMC) to an H-CMC (hydrogen-type CM-cellulose). 1.5 to 2.0 g of the H-CMC that is absolutely dried is precisely weighed and put in a stoppered 300-mL conical flask. The H-CMC is moistened with 15 mL of an 80% methanol; and 100 mL of a 0.1N-NaOH is added and shaken at room temperature for 3 hours. By using phenolphthalein as an indicator, the excess NaOH is back titrated with a 0.1N-H₂SO₄ and the degree of carboxymethyl substitution (DS value) is calculated by the following expression: $A = \left[\left(100\times F′-0.1N-{H}_{2}{SO}_{4}\left(mL\right)\times F\right)\times 0.1\right] / \left(a mass of absolutely dry H-CMC \left(g\right)\right)$
Degree of carboxymethyl substitution = 0.162×A/(1-0.058×A)
F': a factor of the 0.1N-H₂SO₄
F: a factor of the 0.1N NaOH

### <Degree of crystallinity of the cellulose type I>

The degree of crystallinity of cellulose type I in the CM-CNF is, from the viewpoint of improving the foaming performance and the foam stability, 40% or higher, preferably 50% or higher and more preferably 60% or higher. The degree of crystallinity of cellulose type I in the CM-CNFs can be controlled by the concentration of the mercerizing agent in producing the CM-cellulose that is a raw material, the temperature in the treatment, and the degree of carboxymethylation. Since a high-concentration alkali is used in the mercerization and the carboxymethylation, the type I crystal of the cellulose is easily converted to type II; but for example, by regulating the amount of the alkali (mercerizing agent) to be used to regulate the degree of modification, a desired crystallinity can be retained. The upper limit of the degree of crystallinity of cellulose type I is not particularly limited. It is conceivable that practically, the upper limit is about 90%. The degree of crystallinity of cellulose type I of the CM- cellulose and the degree of crystallinity of cellulose type I of the CM-CNFs obtained by defibrating the CM-cellulose are usually the same.

A measuring method of the degree of crystallinity of cellulose type I is as follows.

A sample is mounted on a glass cell, and measured by using an X-ray diffractometer (LabX XRD-6000, manufactured by Shimadzu Corp.). The calculation of the degree of crystallinity is carried out by using means by the Segal et al. method; and the degree of crystallinity is calculated by the following formula from a diffraction intensity of the 002 plane at 2θ = 22.6° and a diffraction intensity of an amorphous portion at 2θ = 18.5°, using a diffraction intensity at 2θ = 10° to 30° in an X-ray diffraction diagram as a base line,: $Xc = \left(I 002c-Ia\right) / I 002 c \times 100$
Xc: a degree of crystallinity of cellulose type I (%)
I 002c: a diffraction intensity of the 002 plane at 2θ = 22.6°
Ia: a diffraction intensity of an amorphous portion at 2θ = 18.5°

### <Nonionic water-soluble polymer and optional surfactant>

By mixing the above CM-CNFs with a nonionic water-soluble polymer and optionally a surfactant, wherein the nonionic water-soluble polymer is at least one selected from hydroxyethylcellulose, methylcellulose, hydroxypropylcellulose, hydroxypropylmethylcellulose, or hydroxyethylmethylcellulose, the additive used in the present invention for producing an air bubble-containing composition is produced. Both of the nonionic water-soluble polymer and the surfactant may be contained in the additive used in the present invention. The nonionic water-soluble polymer for use in the additive used in the invention is at least one selected from hydroxyethylcellulose, methylcellulose, hydroxypropylcellulose, hydroxypropylmethylcellulose and hydroxyethylmethylcellulose. Further, there may be used nonionic water-soluble polymers such as polyethylene glycol, polyvinylpyrollidone, copolymers of vinylpyrollidone and vinyl acetate, galactan, pullulan, mannan, sclerotium gum and guar gum. The nonionic water-soluble polymer may be used by mixing a plurality of the above examples, or may be used singly. Among these, preferable are hydroxyethylcellulose, methylcellulose and polyethylene glycol. Then, examples of the surfactant include, though not limited to, sodium laurylsulfate, lauramidopropyl hydroxysultaine, decyldimethylamine oxide, lauramine oxide, sodium oleate, sodium laureth sulfate, sodium cocoylmethyltaurine, sodium cocoylglutamate, potassium laurate, cocamidopropyl betaine, cocamidodiethanolamide, lauramidopropyl betaine, hydrogenated lecithin and sodium cocoamphoacetate; among these, preferable are sodium laurylsulfate, lauramidopropyl hydroxysultaine, decyldimethylamine oxide, lauramine oxide and sodium oleate, and more preferable are sodium laurylsulfate, lauramidopropyl hydroxysultaine and decyldimethylamine oxide. The surfactant may be used by mixing a plurality of the above examples, or may be used singly. Specific kinds of the nonionic water-soluble polymer and the surfactant may suitably be selected according to an air bubble (foam)-containing composition to be produced.

With regard to the mixing proportion of the CM-CNFs and the nonionic water-soluble polymer, the amount of the nonionic water-soluble polymer is preferably 1 to 5,000 parts by mass, with respect to 100 parts by mass of the amount of the CM-CNFs. The amount is more preferably 5 to 5,000 parts by mass, still more preferably 5 to 2,000 parts by mass, further still more preferably 5 to 1,000 parts by mass, further still more preferably 10 to 1,000 parts by mass, further still more preferably 20 to 1,000 parts by mass and further still more preferably 20 to 500 parts by mass. Here, any of amounts of the CM-CNFs and the nonionic water-soluble polymer is an amount as a solid content.

With regard to the mixing proportion of the CM-CNFs and the nonionic water-soluble polymer and optional surfactant, the amount of the nonionic water-soluble polymer and optional surfactant is preferably 1 to 5,000 parts by mass, with respect to 100 parts by mass of the amount of the CM-CNFs. The amount is more preferably 5 to 5,000 parts by mass, still more preferably 5 to 2,000 parts by mass, further still more preferably 5 to 1,000 parts by mass, further still more preferably 10 to 1,000 parts by mass, further still more preferably 20 to 1,000 parts by mass and further still more preferably 20 to 500 parts by mass. The "amount of the nonionic water-soluble polymer and optional surfactant" is, in the case of using both of the nonionic water-soluble polymer and optional surfactant, a total amount thereof. Here, any of amounts of the CM-CNFs, the nonionic water-soluble polymer and optional surfactant is an amount as a solid content

A method of mixing the CM-CNFs with the nonionic water-soluble polymer and optional surfactant is not particularly limited; for example, dispersion in which one of them is dispersed in a disperse medium such as water and dispersion in which another of them is dispersed similarly may be mixed; or one of them in the form of powder or a solid may be added to and mixed with dispersion in which another of the is dispersed.

### <Additive for use in producing an air bubble-containing composition>

As described above, an additive for use in producing an air bubble-containing composition is produced by mixing the CM-CNFs having a specific degree of carboxymethyl substitution and a specific degree of crystallinity of cellulose type I, with the nonionic water-soluble polymer and optional surfactant. The "air bubble-containing composition" (or the foam-containing composition) refers to a composition containing a gas such as air in a foam state. The air bubble-containing composition is usually formed by stirring a liquid with a stirrer, a whisk or the like. Examples of applications of the air bubble-containing composition include, though not limited to, foods and beverages (ice cream, dough for cakes, hot cakes and the like, bread, whipped cream, and the like), cosmetics (facial wash, shaving cream and the like), detergents (solid soaps, kitchen detergents, body soaps, shampoos, tooth powders and the like), resins, agrochemicals, civil engineering materials, boring materials (foam shielding materials and the like), and the like, which are intended to contain foam or to cause foaming in use.

The "additive for use in producing an air bubble-containing composition " includes an additive to be mixed in materials for the air bubble-containing composition (components of a composition before foam (or air bubble) is formed) when producing the air bubble-containing composition, and an additive to be added to the air bubble-containing composition. In the former case, by carrying out stirring or the like after addition of the additive, the materials for the air bubble-containing composition cause foaming to form the air bubble-containing composition. In the latter case, by carrying out stirring or the like after addition of the additive, the additive is mixed homogeneously in the composition. In either case, the foaming performance (power of foam formation) and the foam stability (power of formed foam retention) can be imparted to the composition.

In the case of adding the additive for use in producing an air bubble-containing composition to materials for the air bubble-containing composition (components of the composition before foam is formed), the materials for the air bubble-containing composition may contain all components to be contained in a final air bubble-containing composition, or may contain a part of components to be contained in a final air bubble-containing composition. In the latter case, after addition of the additive used in the present invention, and after or while the materials for the air bubble-containing composition (containing a part of components of the air bubble-containing composition) cause foaming, the residual components of the composition may be mixed. The materials for the air bubble-containing composition to which the additive used in the present invention is to be added may contain in advance a component corresponding to a foaming agent to promote the formation of foam and prevent the formed foam from merging or disappearing. Examples of the foaming agent include surfactants, for example, higher-alcohol sulfate ester salts, alkyl ether sulfate ester salts, N-acylglutamate salts and phosphate ester salts, and besides, proteins such as collagen and keratin, and cellulose compounds such as carboxymethyl cellulose and crystalline cellulose.

The form of the additive for use in producing an air bubble-containing composition is not particularly limited. The form may be, for example, a form of a dispersion which contains the CM-CNFs, the nonionic water-soluble polymer and/or the surfactant, and water or the like as a disperse medium, a form of a dry solid made by drying such a dispersion, or a form of a powder made by pulverizing the dry solid. The form may also be a form of a wet solid material in an intermediate state between the dispersion and the dry solid. In the present description, the dry solid refers to one made by deliquoring and drying a dispersion so that the amount of liquid such as water is 12% by mass or smaller. The amount of the liquid in the dry solid can be measured by the following method:
The dry solid is dried in an oven at 105°C for 12 hours; and the amount of liquid in the dry solid is calculated from masses thereof before and after the drying. 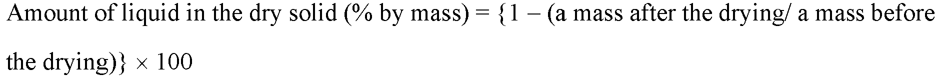 Amount of liquid in the dry solid (% by mass) = {1 - (a mass after the drying/ a mass before the drying)} × 100

In the case where the additive for use in producing an air bubble-containing composition is in the form of a dispersion, the disperse medium is preferably water or a mixed solvent of water and a hydrophilic organic solvent, in consideration of the dispersibility of the CM-CNFs. The disperse medium in the production of the CM-CNFs may be used as it is, or the disperse medium containing a hydrophilic organic solvent may be prepared by adding a hydrophilic organic solvent, or by replacing a part of the disperse medium with a hydrophilic organic solvent. In the case of using a disperse medium containing both of water and a hydrophilic organic solvent, the amount of the hydrophilic organic solvent is preferably 10% by mass or larger, more preferably 20% by mass or larger and still more preferably 25% by mass or larger, with respect to the total amount of water and the hydrophilic organic solvent. The upper limit of the amount is not limited, but is preferably 95% by mass or smaller and more preferably 80% by mass or smaller.

The hydrophilic organic solvent refers to an organic solvent which dissolves in water. Examples thereof include, though not limited to, methanol, ethanol, 2-propanol, butanol, glycerol, butylene glycol, propanediol, acetone, methyl ethyl ketone, 1,4-dioxane, N-methyl-2-pyrollidone, tetrahydrofuran, N,N-dimethylforamide, N,N-dimethylacetoamide, dimethyl sulfoxide, acetonitrile, and combinations thereof. Among these, preferable are lower alcohols having 1 to 4 carbon atoms, such as methanol, ethanol and 2-propanol, and polyols such as glycerol, butylene glycol and propanediol; and from the viewpoint of the safety and the easy availability, preferable are methanol, ethanol, glycerol, butylene glycol and propanediol.

In the case where the additive for use in producing an air bubble-containing composition is in the form of a dry solid, it is preferable that a pH of a dispersion of the CM-CNFs and the nonionic water-soluble polymer and/or the surfactant is regulated to 9 to 11, and then, is deliquored and dried. When the dispersion is dried after the pH is regulated to 9 to 11, dispersibility of the additive to materials for the air bubble-containing composition or to the air bubble-containing composition becomes good. In the regulation of the pH, an alkali such as sodium hydroxide may be used.

As a method of deliquoring and drying the dispersion, a known method can be used; and examples thereof include spray drying, compression, air-drying, hot-air drying and vacuum drying. A drying apparatus is not particularly limited, but there can be used a continuous tunnel dryer, band dryer, vertical dryer, vertical turbo-dryer, multi-stage disk dryer, through-flow dryer, rotary dryer, pneumatic conveying dryer, spray dryer, atomizer, cylinder dryer, drum dryer, belt dryer, screw conveyer dryer, heating tube-equipped rotary dryer, vibration conveyor dryer, batch-type box dryer, through-flow dryer, vacuum box dryer or an agitated dryer, singly, or a combination of two or more thereof.

Among these, use of an apparatus which forms a thin film and dries the film is preferable from the viewpoint of the energy efficiency because the apparatus can homogeneously supply heat energy directly to a material to be dried, and can carry out the drying treatment more efficiently in a short time. Then, the apparatus forming a thin film and drying the film is preferable also from the viewpoint that a dry material can immediately be recovered by simple means such as scraping-off of the thin film, and is preferable also from the viewpoint of improving the dispersibility of an obtained dry solid in materials for the air bubble-containing composition or in the air bubble-containing composition. Examples of the apparatus forming a thin film and drying the film include a drum dryer and a belt dryer in which a thin film is formed on a drum or a belt and is dried. Among these, the drum dryer is preferable since continuous drying and recovery of a dry material are easily conducted.

The drum dryer is an apparatus which continuously supplies a dispersion on the drum surface under rotation of the heated drum, deposits the dispersion in the form of a thin film on the drum surface by evaporating and concentrating the disperse medium, dries the film, and scrapes a dry material formed on the drum surface by a knife to produce a dry solid. The drum dryer includes a double drum type or twin drum type using two drums, and a single drum type using one drum, and either thereof may be used. Among these, preferable is a double drum-type apparatus which can regulate the film thickness by regulating the clearance between the drums.

The thickness of the thin film to be dried is preferably 50 to 1,000 µm and more preferably 100 to 300 µm. When the thickness is 50 µm or larger, the scraping-off after the drying is easy; and when smaller than 1,000 µm, there is observed the effect of improving the dispersibility in materials for the air bubble-containing composition or in the air bubble-containing composition.

The drying temperature is not particularly limited. The drying can be carried out at a temperature of, for example, about 200°C or lower. In the drying in a drum dryer or on a belt dryer for forming and drying the thin film, the drying temperature refers to the temperature of the drum or belt surface.

The drying may be carried out under ordinary pressure, or under vacuum or reduced pressure. Among these, carrying out the drying under vacuum or reduced pressure is preferable because there are attained advantages of lowering the boiling point of moisture, accelerating vaporization speed, promoting drying of an object and reducing the thermal influence on a sample.

In the drying under vacuum or reduced pressure (hereinafter, referred to also as "vacuum drying"), it is preferable that the drying is carried out in the range of 0 to 50 kPa. Since a lower pressure can vaporize moisture at a lower temperature, the pressure in the drying is preferably 50 kPa or lower, more preferably 30 kPa or lower and still more preferably 10 kPa or lower.

It is preferable to obtain the dry solid by vacuum drying the dispersion at a relatively low temperature of 40 to 100°C in the drying. Since a low drying temperature decreases production efficiency, the drying temperature is preferably 40°C or higher, more preferably 45°C or higher and still more preferably 50°C or higher. On the other hand, since a high drying temperature causes coloration and damage of the cellulose, the drying temperature is preferably 100°C or lower, more preferably 90°C or lower, more preferably 85°C or lower and still more preferably 80°C or lower, and may be lower than 80°C.

The vacuum dryer is not particularly limited, but there can be used a box-type vacuum dryer, a drum-type vacuum dryer, a spray-type vacuum dryer or a belt-type vacuum dryer, singly, or a combination of two or more. The vacuum drum dryer is an apparatus which continuously supplies the dispersion on the drum surface under rotation of the heated drum placed under vacuum or reduced pressure, evaporates and concentrates a disperse medium to deposit the dispersion in the form of a thin film on the drum surface, dries the film, and scrapes a dry material formed on the drum surface by a knife to produce a dry solid.

When the obtained dry solid is pulverized to make a powder, the median diameter of the powder is preferably about 10.0 to 150.0 µm, more preferably 25.0 to 100.0 µm and still more preferably 35.0 to 70.0 µm. When the median diameter is 10.0 µm or larger, problems such as powder scattering hardly arise; and when it is 150.0 µm or smaller, since the powder has a suitable bulk, packing of the powder becomes easy. The median diameter of the powder can be regulated by adjusting the conditions of pulverization and classification. Here, the median diameter can be measured by the following procedure:
Methanol is used as a disperse medium and a sample is prepared so that the scattered intensity is 0.1 to 20%; and the median diameter is measured by a laser diffraction particle size analyzer (manufactured by Malvern Panalytical Ltd., Mastersizer(R) 3000).

### <Addition to materials for the air bubble-containing composition or to the air bubble-containing composition>

The additive used in the present invention for producing an air bubble-containing composition is mixed with materials for the air bubble-containing composition (components of the composition before foam is formed), and stirred for foaming the materials, whereby the air bubble (foam)-containing composition can be produced. The additive used in the present invention can impart good foaming performance to the materials for the air bubble-containing composition and enhance the persistency of foam formed (foam stability). The materials for the air bubble-containing composition may contain all components to be contained in the final air bubble-containing composition, or may contain a part of components to be contained in the final air bubble-containing composition. In the latter case, after the addition of the additive used in the present invention, the residual components of the composition may be mixed after or while the materials for the air bubble-containing composition (a part of components of the foam-containing composition) cause foaming.

By adding the additive used in the present invention for producing an air bubble-containing composition to the air bubble (foam)-containing composition, foaming can further be promoted and the foam stability can be enhanced.

It is preferable that after the additive used in the present invention is added to the materials for the air bubble-containing composition or to the air bubble-containing composition, foaming is promoted by stirring using a known mixing and stirring apparatus. The conditions of the stirring may suitably be determined according to the kind of the air bubble-containing composition and a desired degree of foaming and is not particularly limited, but the stirring may be carried out, for example, at about 100 to 15,000 rpm, preferably about 500 to 10,000 rpm, for about 1 to 30 min, preferably about 1 to 15 min.

The content of the additive used in the present invention in the final air bubble-containing composition may suitably be varied according to the kind of the air bubble-containing composition and a desired degree of foaming and is not particularly limited. It is preferable that the addition is made, for example, so that the amount of the CM-CNFs derived from the additive is 0.05% by mass or larger with respect to the amount of the air bubble-containing composition; and it is more preferable that the addition is made, for example, so that the amount is 0.1% by mass or larger.

### EXAMPLES

Hereinafter, the present invention will be described in more detail by way of Examples, but the present invention is not any more limited thereto. It is noted that neither the reference examples nor the comparative examples are according to the invention.

### <Producing CM-CNFs>

In a twin screw kneader regulated at a rotating speed of 100 rpm, there was added a solution in which 620 parts of isopropanol (IPA) and 10 parts of sodium hydroxide were dissolved in 30 parts of water, and there was charged 100 parts of a hardwood pulp (LBKP)(manufactured by Nippon Paper Industries Co., Ltd., LBKP) in dry mass when being dried at 100°C for 60 min. The resultant was stirred and mixed at 30°C for 90 min to thereby prepare a mercerized cellulose. Further under stirring, 15 parts of IPA and 12 parts of monochloroacetic acid were added, and stirred for 30 min, and thereafter, the temperature was raised to 70°C to carry out a carboxymethylation reaction for 90 min. After the finish of the reaction, the resultant was neutralized with acetic acid until the pH became 7, and cleaned with hydrous methanol, then deliquored, dried and pulverized to thereby obtain a CM-cellulose having a degree of carboxymethyl substitution of 0.18.

The CM-cellulose obtained in the above step was adjusted to 1.0% by mass by water, and three times treated by a ultrahigh-pressure homogenizer (20°C, 150 MPa) to thereby obtain a dispersion of CM-CNFs. The obtained fibers had an average fiber diameter of 5 nm, an aspect ratio of 150 and a degree of crystallinity of cellulose type I of 72%.

### <Example 1>

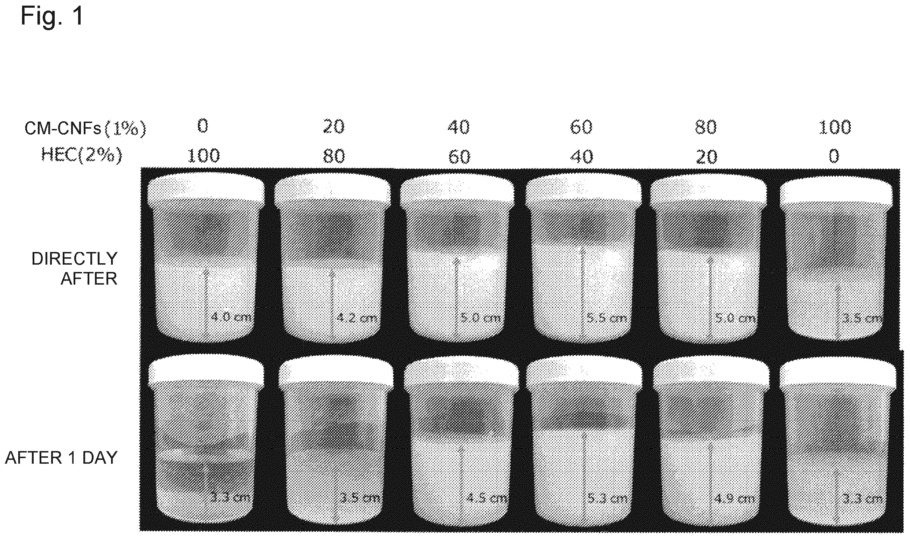

There was prepared a 1.0-mass% water dispersion of the CM-CNFs obtained in the above. A hydroxyethylcellulose (manufactured by Daicel Corp., trade name: SE550) (hereinafter, "hydroxyethylcellulose" is abbreviated as "HEC") was selected as a nonionic water-soluble polymer, and there was prepared a 2.0-mass% water dispersion of the HEC so that the viscosity of the HEC water dispersion became nearly equal to that of the CM-CNFs 1.0-mass% water dispersion. The obtained CM-CNFs 1.0-mass% water dispersion and HEC 2.0-mass% water dispersion were mixed so as to achieve the mass ratios described in Table 1 in 250 ml-volume capped polypropylene vessels (manufactured by As One Corporation, trade name: Pack Clean). Each sample was stirred in a Homo Disper at 3,000 rpm for 10 min. In order to evaluate the foaming performance and the foam stability, the heights from the bottom surface of the vessel to the upper surface of the dispersion were measured both directly after the stirring and after still standing at room temperature for 1 day. Photographs were also taken. The photographs are shown in Fig. 1.

**[Table 1]**

| Sample No. | | 1 | 2 | 3 | 4 | 5 | 6 |
|---|---|---|---|---|---|---|---|
| Mixing Proportion of Water Dispersion | CM-CNFs(1%) | 0 | 20 | 40 | 60 | 80 | 100 |
| | HEC(2%) | 100 | 80 | 60 | 40 | 20 | 0 |
| Solid Content (%) | CM-CNFs | 0 | 0.2 | 0.4 | 0.6 | 0.8 | 1.0 |
| | HEC | 2.0 | 1.6 | 1.2 | 0.8 | 0.4 | 0 |
| Proportion of HEC (parts by mass) (with respect to 100 parts by mass of CM-CNFs) | | - | 800 | 300 | 133 | 50 | 0 |
| Height of Dispersion (cm) | directly after stirring | 4.0 | 4.2 | 5.0 | 5.5 | 5.0 | 3.5 |
| | after 1 day | 3.3 | 3.5 | 4.5 | 5.3 | 4.9 | 3.3 |

From the photographs of Fig. 1, in the case of HEC alone, foaming was confirmed directly after the stirring. However, it is found that the formed foam almost disappeared after the still standing for 1 day. On the other hand, in the case of the CM-CNFs alone, it is found that the foaming performance directly after the stirring was low. By contrast, in any of the samples in which HEC and CM-CNFs were mixed, it is found that the foaming performance and the foam stability were improved.

For samples in which the obtained CM-CNFs 1.0-mass% water dispersion and HEC 2.0-mass% water dispersion were mixed in mass ratios in the range of 90 : 10 to 30 : 70 (that is, in the range of 22 to 467 parts by mass of HEC with respect to 100 parts by mass of the CM-CNFs) which were put in vials and allowed to stand still at room temperature, it was confirmed that foam remained even after one week. From the above, it has been found that by mixing and using the HEC and the CM-CNFs, the initial high foaming performance and the high foam stabilizing effect with time can be obtained.

### <Reference Example 2>

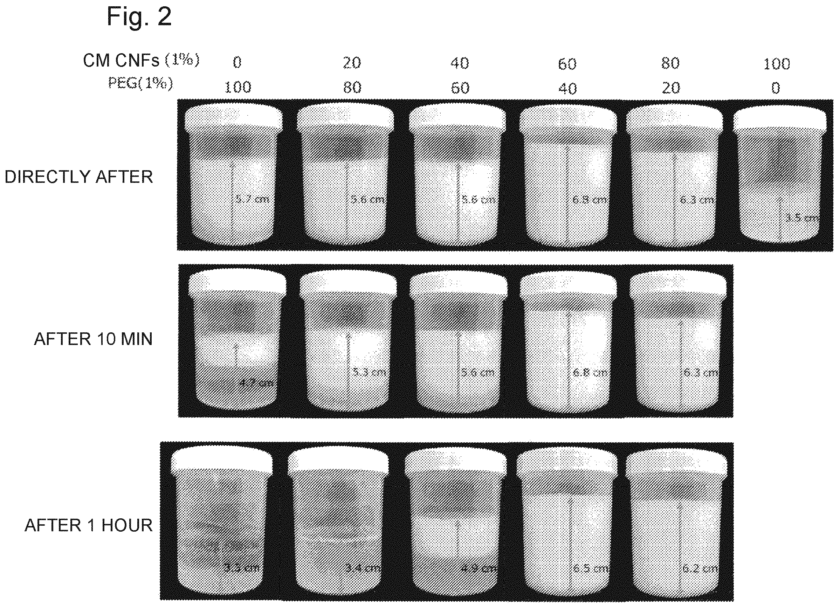

There was prepared a 1.0-mass% water dispersion of the CM-CNFs obtained in the above. A 1.0-mass% water dispersion of a polyethylene glycol (molecular weight: 4,000,000) (hereinafter, "polyethylene glycol" is abbreviated as "PEG") as a nonionic water-soluble polymer was prepared. The obtained CM-CNFs 1.0-mass% water dispersion and PEG 1.0-mass% water dispersion were added so as to achieve the mass ratios described in Table 2 in 250 ml-volume capped polypropylene vessels (manufactured by As One Corporation, trade name: Pack Clean) for 100g each. Each sample was stirred in a Homo Disper at 3,000 rpm for 10 min. In order to evaluate the foaming performance and the foam stability, the heights from the bottom surface of the vessel to the upper surface of the dispersion were determined for the samples directly after the stirring, after still standing at room temperature for 10 min, and after still standing at room temperature for 1 hour. Photographs were also taken. The photographs are shown in Fig. 2.

**[Table 2]**

| Sample No. | | 7 | 8 | 9 | 10 | 11 | 12 |
|---|---|---|---|---|---|---|---|
| Mixing Proportion of Water Dispersion | CM-CNFs(1%) | 0 | 20 | 40 | 60 | 80 | 100 |
| | PEG(1%) | 100 | 80 | 60 | 40 | 20 | 0 |
| Solid Content (%) | CM-CNFs | 0 | 0.2 | 0.4 | 0.6 | 0.8 | 1.0 |
| | PEG | 1.0 | 0.8 | 0.6 | 0.4 | 0.2 | 0 |
| Proportion of PEG (parts by mass) (with respect to 100 parts by mass of CM-CNFs) | | - | 400 | 150 | 67 | 25 | 0 |
| Height of Dispersion (cm) | directly after stirring | 5.7 | 5.6 | 5.6 | 6.8 | 6.3 | 3.5 |
| | after 10 min | 4.7 | 5.3 | 5.6 | 6.8 | 6.3 | not measured |
| | after 1 hour | 3.3 | 3.4 | 4.9 | 6.5 | 6.2 | not measured |

From the photographs of Fig. 2, in the case of PEG alone, foaming can be confirmed directly after the stirring, but it can be seen that defoaming progressed after 10 min. By contrast, in any of the samples in which PEG and CM-CNFs were mixed, it can be seen that the foaming performance and the foam stability were both improved.

### <Comparative Example 1>

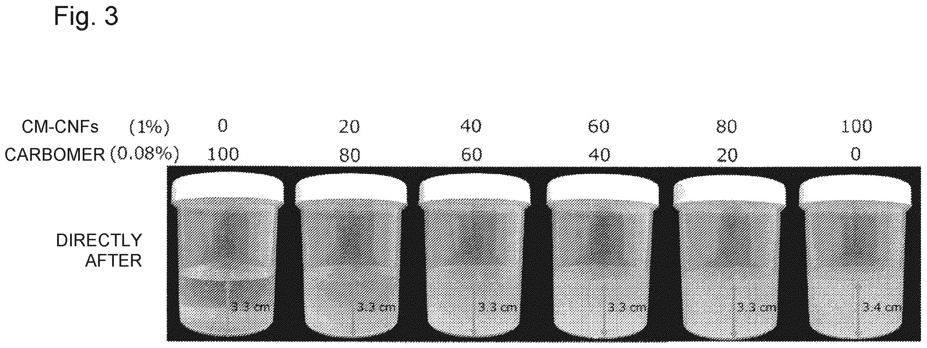

There was prepared a 1.0-mass% water dispersion of the CM-CNFs obtained in the above. A carboxyvinyl polymer (manufactured by Iwase Cosfa Co., Ltd., trade name: Carbopol(R) 940Polymer) (hereinafter, "carboxyvinyl polymer" is abbreviated as "carbomer") was used as an anionic water-soluble polymer, and a 0.08-mass% water dispersion of the carbomer was prepared so that the viscosity of the carbomer water dispersion became nearly equal to that of the CM-CNFs 1.0-mass% water dispersion. The obtained CM-CNFs 1.0-mass% water dispersion and carbomer 0.08-mass% water dispersion were added so as to achieve the mass ratios described in Table 3 in 250 ml-volume capped polypropylene vessels (manufactured by As One Corporation, trade name: Pack Clean) 100 g each. Each sample was stirred in a Homo Disper at 3,000 rpm for 10 min. For the samples directly after the stirring, the heights from the bottom surface of the vessel to the upper surface of the dispersion were measured, as in Examples 1 and 2. Photographs were also taken. The photographs are shown in Fig. 3.

**[Table 3]**

| Sample No. | | 13 | 14 | 15 | 16 | 17 | 18 |
|---|---|---|---|---|---|---|---|
| Mixing Proportion of Water Dispersion | CM-CNFs(1%) | 0 | 20 | 40 | 60 | 80 | 100 |
| | carbomer (0.08%) | 100 | 80 | 60 | 40 | 20 | 0 |
| Solid Content (%) | CM-CNFs | 0 | 0.2 | 0.4 | 0.6 | 0.8 | 1.0 |
| | carbomer | 0.08 | 0.06 | 0.05 | 0.03 | 0.02 | 0 |
| Proportion of Carbomer (parts by mass) (with respect to 100 parts by mass of CM-CNFs) | | - | 32 | 12 | 5 | 2 | 0 |
| Height of Dispersion (cm) | directly after stirring | 3.3 | 3.3 | 3.3 | 3.3 | 3.3 | 3.4 |

From the photographs of Fig. 3, it is found that in either of the case of the carbomer alone and the case where the carbomer and the CM-CNFs were mixed, almost no foaming occurred and the foaming performance was low.

### <Comparative Example 2>

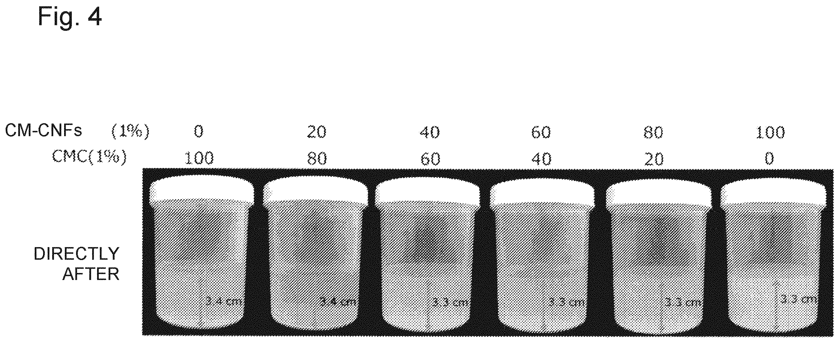

A carboxymethylcellulose (manufactured by Nippon Paper Industries Co., Ltd., F350HC-4)(hereinafter, "carboxymethylcellulose" is abbreviated as "CMC") being an anionic water-soluble polymer was replaced for PEG in Example 2, and as in Example 2, a CM-CNFs 1.0-mass% water dispersion and a CMC 1.0-mass% water dispersion were mixed so as to achieve the mass ratios described in Table 4 and put in polypropylene vessels. Each sample was stirred in a Homo Disper at 3,000 rpm for 10 min. For the samples directly after the stirring, the heights from the bottom surface of the vessel to the upper surface of the dispersion were measured, as in Examples 1 and 2. Photographs were also taken. The photographs are shown in Fig. 4.

**[Table 4]**

| Sample No. | | 19 | 20 | 21 | 22 | 23 | 24 |
|---|---|---|---|---|---|---|---|
| Mixing Proportion of Water Dispersion | CM-CNFs(1%) | 0 | 20 | 40 | 60 | 80 | 100 |
| | CMC(1%) | 100 | 80 | 60 | 40 | 20 | 0 |
| Solid Content (%) | CM-CNFs | 0 | 0.2 | 0.4 | 0.6 | 0.8 | 1.0 |
| | CMC | 1.0 | 0.8 | 0.6 | 0.4 | 0.2 | 0 |
| Proportion of CMC (parts by mass) (with respect to 100 parts by mass of CM-CNFs) | | - | 400 | 150 | 67 | 25 | 0 |
| Height of Dispersion (cm) | directly after stirring | 3.4 | 3.4 | 3.3 | 3.3 | 3.3 | 3.3 |

From the photographs of Fig. 4, it is found that in either of the case of the CMC alone and the case where the CMC and the CM-CNFs were mixed, almost no foaming occurred and the foaming performance was low.

### <Comparative Example 3>

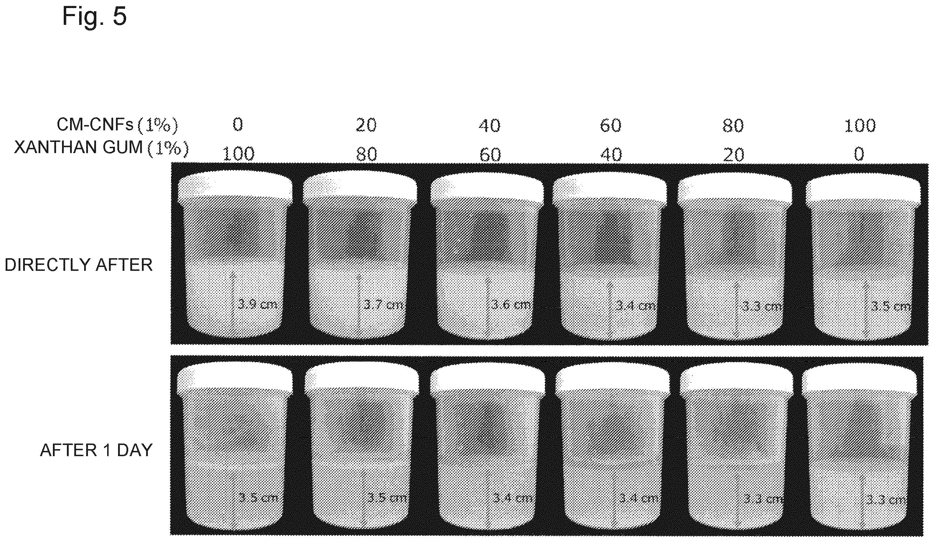

A xanthan gum (manufactured by Sansho Co., Ltd., Keltrol(R) CG-T) being an anionic water-soluble polymer was replaced for PEG in Example 2, and as in Example 2, a CM-CNFs 1.0-mass% water dispersion and a xanthan gum 1.0-mass% water dispersion were mixed so as to achieve the mass ratios described in Table 5 and put in polypropylene vessels, and each sample was stirred in a Homo Disper at 3,000 rpm for 10 min. For the samples directly after the stirring, and after 1 day, the heights from the bottom surface of the vessel to the upper surface of the dispersion were measured, as in Examples 1 and 2. Photographs were also taken. The photographs are shown in Fig. 5.

**[Table 5]**

| Sample No. | | 25 | 26 | 27 | 28 | 29 | 30 |
|---|---|---|---|---|---|---|---|
| Mixing Proportion of Water Dispersion | CM-CNFs(1%) | 0 | 20 | 40 | 60 | 80 | 100 |
| | xanthan gum (1%) | 100 | 80 | 60 | 40 | 20 | 0 |
| Solid Content (%) | CM-CNFs | 0 | 0.2 | 0.4 | 0.6 | 0.8 | 1.0 |
| | xanthan gum | 1.0 | 0.8 | 0.6 | 0.4 | 0.2 | 0 |
| Proportion of Xanthan Gum (parts by mass) (with respect to 100 parts by mass of CM-CNFs) | | - | 400 | 150 | 67 | 25 | 0 |
| Height of Dispersion (cm) | directly after stirring | 3.9 | 3.7 | 3.6 | 3.4 | 3.3 | 3.5 |
| | after 1 day | 3.5 | 3.5 | 3.4 | 3.4 | 3.3 | 3.3 |

From the photographs of Fig. 5, in the case of the xanthan gum alone, foaming can be confirmed directly after the stirring. However, it can be seen that foam disappeared after 1 day. Further, also in the samples in which the xanthan gum and the CM-CNFs were mixed, foaming can be confirmed directly after the stirring, but no clear improvement in foaming performance was observed and it can be seen that foam disappeared after 1 day as in the case of the xanthan gum alone.

### <Reference Example 3>

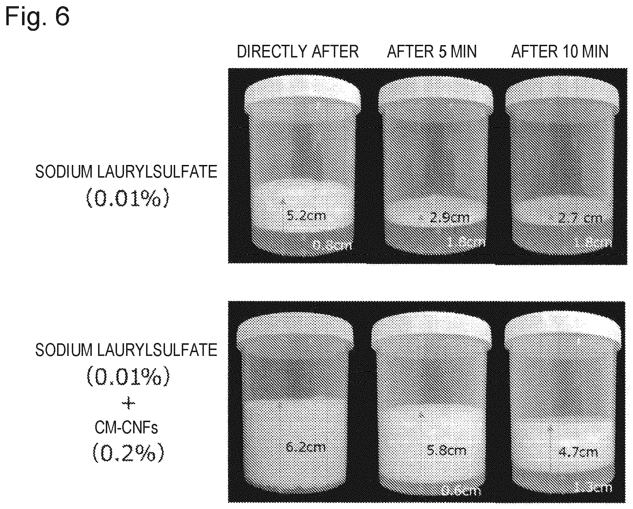

100 g of a water dispersion containing 0.2% by mass of the CM-CNFs obtained in the above and 0.01% by mass of sodium laurylsulfate was put in a 600 ml-volume capped polypropylene vessel (manufactured by As One Corporation, trade name: Pack Clean), and was stirred in a Homo Disper at 3,000 rpm for 5 min. As comparison, also a water dispersion containing 0.01% by mass of sodium laurylsulfate alone was similarly put in a vessel and stirred. In order to evaluate the foaming performance and the foam stability, the heights from the bottom surface of the vessel to the upper surface of the dispersion were measured for the samples directly after the stirring, after still standing at room temperature for 5 min, and after still standing at room temperature for 10 min. Photographs were also taken. The photographs are shown in Fig. 6.

From the photographs of Fig. 6, it can be seen that in the case of sodium laurylsulfate alone, the separation of a foam layer and a liquid phase was observed directly after the stirring. On the other hand, it can be seen that in the sample in which sodium laurylsulfate and the CM-CNFs were mixed, the foaming performance directly after the stirring was improved and the foam stability with time was also improved.

### <Example 4>

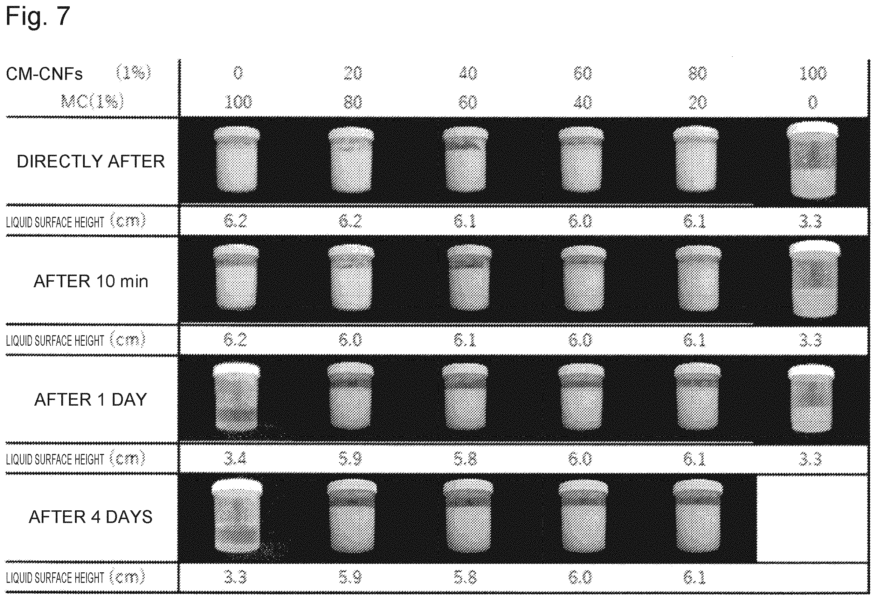

There was prepared a 1.0-mass% water dispersion of the CM-CNFs obtained in the above. Then, a 1.0-mass% water dispersion of a methylcellulose (Methyl Cellulose (7,000 to 10,000 mPa·s, 2% in water at 20°C), Tokyo Chemical Industry Co., Ltd.)(hereinafter, "methylcellulose" is abbreviated as "MC") as a nonionic water-soluble polymer was prepared. The obtained CM-CNFs 1.0-mass% water dispersion and MC 1.0-mass% water dispersion were added 100 g each so as to achieve the mass ratios described in Table 6 in 250 ml-volume capped polypropylene vessels (manufactured by As One Corporation, trade name: Pack Clean). Each sample was stirred in a Homo Disper at 3,000 rpm for 5 min. In order to evaluate the foaming performance and the foam stability, the heights from the bottom surface of the vessel to the upper surface of the dispersion were measured for the samples directly after the stirring, after still standing at room temperature for 10 min, after still standing at room temperature for 1 day, and after still standing at room temperature for 4 days. Photographs were also taken. The photographs are shown in Fig. 7.

**[Table 6]**

| Sample No. | | 31 | 32 | 33 | 34 | 35 | 36 |
|---|---|---|---|---|---|---|---|
| Mixing Proportion of Water Dispersion | CM-CNFs(1%) | 0 | 20 | 40 | 60 | 80 | 100 |
| | MC(1%) | 100 | 80 | 60 | 40 | 20 | 0 |
| Solid Content (%) | CM-CNFs | 0 | 0.2 | 0.4 | 0.6 | 0.8 | 1.0 |
| | MC | 1.0 | 0.8 | 0.6 | 0.4 | 0.2 | 0 |
| Proportion of MC (parts by mass) (with respect to 100 parts by mass of CM-CNFs) | | - | 400 | 150 | 67 | 25 | 0 |
| Height of Dispersion (cm) | directly after stirring | 6.2 | 6.2 | 6.1 | 6.0 | 6.1 | 3.3 |
| | after 10 min | 6.2 | 6.0 | 6.1 | 6.0 | 6.1 | 3.3 |
| | after 1 day | 3.4 | 5.9 | 5.8 | 6.0 | 6.1 | 3.3 |
| | after 4 days | 3.3 | 5.9 | 5.8 | 6.0 | 6.1 | not measured |

From the photographs of Fig. 7, in the case of MC alone, foaming can be confirmed directly after the stirring, but it can be seen that the formed foam almost disappeared after the still standing for 1 day. On the other hand, in the case of the CM-CNFs alone, it can be seen that the foaming performance directly after the stirring was low. By contrast, it can be seen that in any of the samples in which MC and CM-CNFs were mixed, the foaming performance and the foam stability were improved.

### <Reference Example 5>

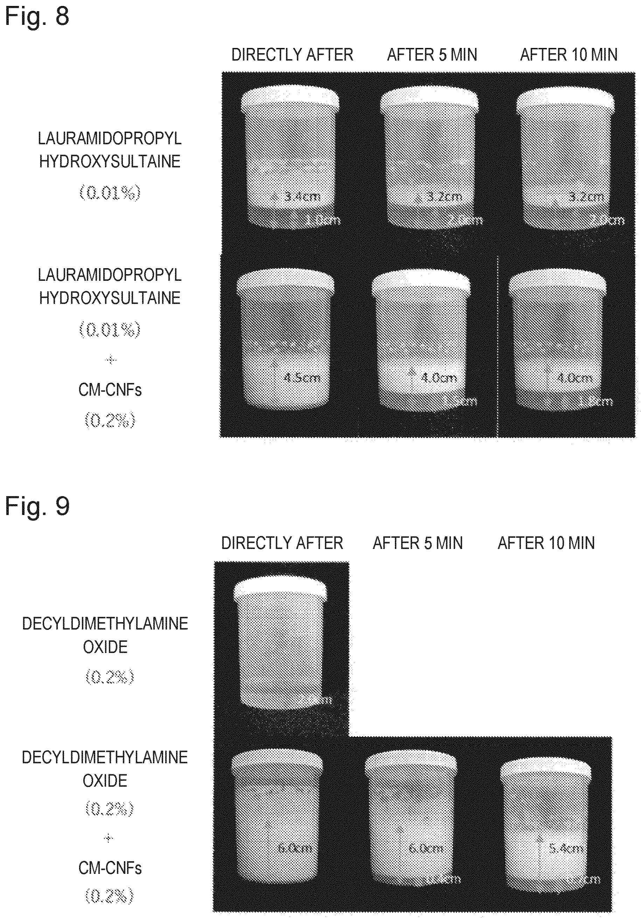

100 g of a water dispersion containing 0.2% by mass of the CM-CNFs obtained in the above and 0.01% by mass of lauramidopropyl hydroxysultaine (Kawaken Fine Chemical Co., Ltd., trade name: Softazoline(R) LSB-R) was put in a 600 ml-volume capped polypropylene vessel (manufactured by As One Corporation, trade name: Pack Clean), and was stirred in a Homo Disper at 3,000 rpm for 5 min. As comparison, also a water dispersion containing 0.01% by mass of lauramidopropyl hydroxysultaine alone was similarly put in a vessel and stirred. In order to evaluate the foaming performance and the foam stability, the heights from the bottom surface of the vessel to the upper surface of the dispersion and the heights from the bottom surface of the vessel to the lower surface of the foam were measure for the samples directly after the stirring, after still standing at room temperature for 5 min, and after still standing at room temperature for 10 min. Photographs were also taken. The photographs are shown in Fig. 8.

From the photograph of Fig. 8, it can be seen that in the case of lauramidopropyl hydroxysultaine alone, the foaming was low and the foam stability was also low. On the other hand, it can be seen that in the sample in which lauramidopropyl hydroxysultaine and the CM-CNFs were mixed, the foaming performance directly after the stirring was improved and the foam stability with time was improved.

### <Reference Example 6>

The foaming performance and the foam stability were evaluated, as in Reference Example 5, except for altering 0.01% by mass of lauramidopropyl hydroxysultaine to 0.2% by mass of decyldimethylamine oxide (Lion Specialty Chemicals Co., Ltd., trade name: Cadenax(R) DM10D-W). The photographs are shown in Fig. 9.

From the photograph of Fig. 9, it is found that in the case of decyldimethylamine oxide alone, no foaming occurred. On the other hand, it is found that by mixing decyldimethylamine oxide and the CM-CNFs, foaming occurred and the foam stability was good.

### <Reference Example 7>

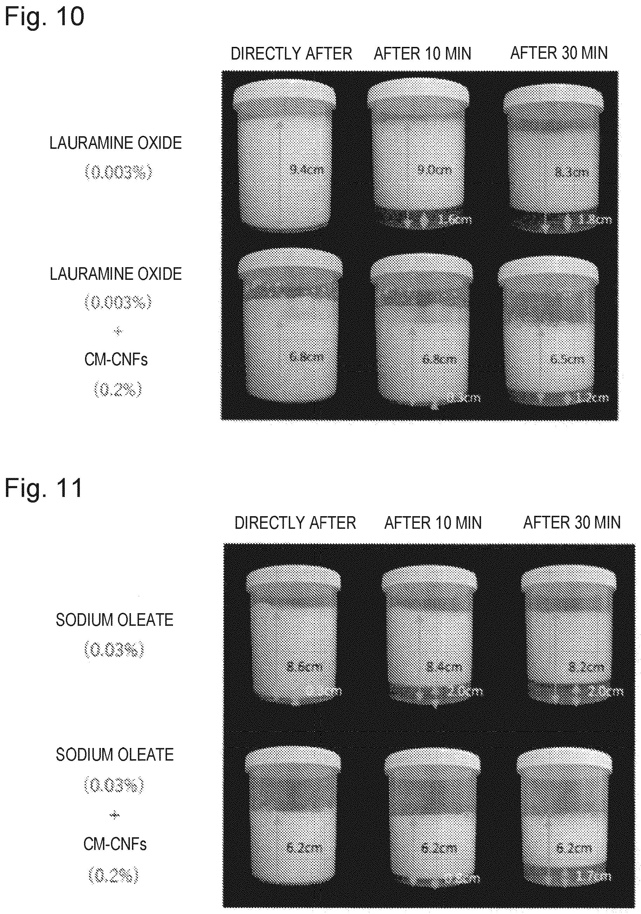

The foaming performance and the foam stability were evaluated, as in Reference Example 5, except for altering 0.01% by mass of lauramidopropyl hydroxysultaine to 0.003% by mass of lauramine oxide (Lion Specialty Chemicals Co., Ltd., trade name: Cadenax(R) DW12D-W(C)). The photographs are shown in Fig. 10.

From the photographs of Fig. 10, it is found that as compared with the case of lauramine oxide alone, in the case where lauramine oxide and the CM-CNFs were mixed, the height from the lower surface of the vessel to the bottom surface of foam hardly increased with time. This indicates that the dispersion in a foam state hardly returned to a liquid state and that mixing lauramine oxide with the CM-CNFs improved the foam stability. Then, as compared with the case of lauramine oxide alone, in the case where lauramine oxide and the CM-CNFs were mixed, finer foam was observed.

### <Reference Example 8>

The foaming performance and the foam stability were evaluated, as in Reference Example 5, except for altering 0.01% by mass of lauramidopropyl hydroxysultaine to 0.03% by mass of sodium oleate. The photographs are shown in Fig. 11

From the photographs of Fig. 11, it is found that as compared with the case of sodium oleate alone, in the case where sodium oleate and the CM-CNFs were mixed, the height from the lower surface of the vessel to the bottom surface of foam hardly increased with time. This indicates that the dispersion in a foam state hardly returned to a liquid state and that mixing sodium oleate with the CM-CNFs improved the foam stability. Then, as compared with the case of sodium oleate alone, in the case where sodium oleate and the CM-CNFs were mixed, finer foam was observed.

## Claims

1. Use of an additive for producing an air bubble-containing composition, the additive comprising:
carboxymethylated cellulose nanofibers having a degree of carboxymethyl substitution per glucose unit of 0.01 to 0.50 and a degree of crystallinity of cellulose type I of 40% or higher; and
a nonionic water-soluble polymer, wherein the nonionic water-soluble polymer is at least one selected from hydroxyethylcellulose, methylcellulose, hydroxypropylcellulose, hydroxypropylmethylcellulose, or hydroxyethylmethylcellulose.

2. The use according to claim 1, wherein an amount of the nonionic water-soluble polymer is 1 to 5,000 parts by mass with respect to 100 parts by mass of the carboxymethylated cellulose nanofibers.

3. A method for producing an air bubble-containing composition, the method comprising mixing and stirring the additive defined in claim 1 or 2 into materials for the air bubble-containing composition to cause foaming of the materials.

4. A method for stabilizing air bubbles in an air bubble-containing composition, comprising adding the additive defined in claim 1 or 2 to the air bubble-containing composition.

## Patentansprüche

1. Verwendung eines Additivs zur Herstellung einer luftblasenhaltigen Zusammensetzung, wobei das Additiv umfasst:
carboxymethylierte Cellulose-Nanofasern mit einem Carboxymethylsubstitutionsgrad pro Glucoseeinheit von 0,01 bis 0,50 und einem Kristallinitätsgrad von Cellulose Typ I von 40 % oder höher; und
ein nichtionisches wasserlösliches Polymer, wobei das nichtionische wasserlösliche Polymer mindestens eines ausgewählt aus Hydroxyethylcellulose, Methylcellulose, Hydroxypropylcellulose, Hydroxypropylmethylcellulose oder Hydroxyethylmethylcellulose ist.

2. Verwendung nach Anspruch 1, wobei eine Menge des nichtionischen wasserlöslichen Polymers 1 bis 5.000 Masseteile, bezogen auf 100 Masseteile der carboxymethylierten Cellulose-Nanofasern, beträgt.

3. Verfahren zur Herstellung einer luftblasenhaltigen Zusammensetzung, wobei das Verfahren Mischen und Rühren des in Anspruch 1 oder 2 definierten Additivs in Materialien für die luftblasenhaltige Zusammensetzung umfasst, um Schäumen der Materialien zu bewirken.

4. Verfahren zum Stabilisieren von Luftblasen in einer luftblasenhaltigen Zusammensetzung, umfassend Zugeben des in Anspruch 1 oder 2 definierten Additivs zu der luftblasenhaltigen Zusammensetzung.

## Revendications

1. Utilisation d'un additif pour la fabrication d'une composition contenant des bulles d'air, l'additif comprenant :
des nanofibres de cellulose carboxyméthylée ayant un degré de substitution par carboxyméthyle par unité glucose de 0,01 à 0,50 et un degré de cristallinité de cellulose de type I de 40 % ou plus ; et
un polymère hydrosoluble non ionique, dans laquelle le polymère hydrosoluble non ionique est au moins l'un choisi parmi l'hydroxyéthylcellulose, la méthylcellulose, l'hydroxypropylcellulose, l'hydroxypropylméthylcellulose ou l'hydroxyéthylméthylcellulose.

2. Utilisation selon la revendication 1, dans laquelle une quantité du polymère hydrosoluble non ionique est de 1 à 5 000 parties en masse par rapport à 100 parties en masse des nanofibres de cellulose carboxyméthylée.

3. Procédé de production d'une composition contenant des bulles d'air, le procédé comprenant le mélange et l'agitation de l'additif défini dans la revendication 1 ou 2 dans des matières pour que la composition contenant des bulles d'air provoque le moussage des matières.

4. Procédé pour stabiliser des bulles d'air dans une composition contenant des bulles d'air, comprenant l'ajout de l'additif défini dans la revendication 1 ou 2 à la composition contenant des bulles d'air.
